# EUROPEAN PATENT SPECIFICATION

(11) **EP 2 744 547 B1**
(45) Date of publication and mention of the grant of the patent: **28.03.2018**
(21) Application number: 12758656.8
(22) Date of filing: 31.08.2012
(51) Int. Cl.: A61M 16/04

(54) **AUTOMATIC TRACHEOSTOMA SPEECH VALVE (ATSV) FIXATION BRACE**
BEFESTIGUNGSORTHESE FÜR TRACHEOSTOMA-SPRACHVENTIL
ORTHÈSE POUR FIXER UNE VALVE PHONATOIRE DE TRACHÉOSTOMIE

(30) Priority: 01.09.2011 NL 2007326
(43) Date of publication of application: 25.06.2014
(73) Proprietor: Stichting Katholieke Universiteit, 6525 GA Nijmegen (NL)
(72) Inventor: DIRVEN, Richard, 6525 EX Nijmegen (NL); BEETZ, Antonius Godefriedus Gerardus, 5708 GT Helmond (NL); MARRES, Henry August Marie, 6525 EX Nijmegen (NL)
(74) Representative: Nederlandsch Octrooibureau
(86) International application number: PCT/NL2012/050600
(87) International publication number: WO 2013/032334

(56) References cited:
- EP-A1- 0 078 685
- WO-A1-2005/046776
- WO-A1-2010/125074
- WO-A2-2007/034493
- DE-A1- 19 807 961
- DE-A1-102006 056 946
- DE-U1-202004 007 566
- DE-U1-202004 020 108
- DE-U1-202006 009 768
- DE-U1-202007 010 008
- DE-U1-202010 004 297
- DE-U1-202010 014 829
- US-A- 4 832 019
- US-A- 5 058 579
- US-A- 5 501 216
- US-A- 5 869 127
- US-A1- 2002 139 372
- US-A1- 2006 005 837

## Description

### Field of the invention

The present invention relates to an automatic tracheostoma speech valve (ATSV) fixation brace.

### Prior art

German patent publication DE-A-10 2006 056946 discloses a device for attaching a tracheal tube to a patient. A soft material type of skin protection is disclosed in combination with a flexible neck band. A hard plastic holder for a cannula is disclosed, also provided with a soft material protection material, but only at the location of the cannula holder. Although the publication mentions thermoplastic material, it is clear that the protection material is actually meant to be soft during actual use (silicone or elastomer material).

German patent publication DE-U-20 2010 004297 discloses a shield for a tracheal cannula, with a flared form and lash eyes for a neck band to ensure proper holding of a tracheal tube. This shield is made of hard material in order to hold the tracheal tube firmly in position, in combination with the position of the neck band lashes.

International patent publication WO2005/046776 discloses a headband for an oxygen mask, part of which is made of foamed plastic material. The disclosure teaches the skilled person to use foamed plastic material for protecting a person's back head against forces acting on the mask.

The publication of German Gebrauchsmuster DE-U-2006 009 768 discloses a device for attaching a tracheal tube. A two part neck band is provided which has end connectors which can be fastened to attachment lips of a tracheal tube. The two parts can be connected around a patient's neck, e.g. using velcro or the like.

American patent publication US 2008/0149107 discloses a holder for a medical tube, such as a tracheal tube. The holder can be fixed around a patient's neck using a flexible neck band, and allows fixation of tubes with varying diameter.

International patent publication WO2008/127189 discloses a device for supporting and retaining medical appliances, such as tracheal cannulas and stoma stents. The device comprises a ring shaped supporting means for fixation of a tube, and an elongated fastening means which can be fixed to a patient's chest.

### Summary of the invention

When a patient has undergone a total laryngectomy, speech remains possible by pressing air through a tracheoesophageal speech valve into the esophagus, which however is only possible when closing the tracheostoma. This can be done manually, or using an automatic tracheostoma speech valve. In existing devices for holding a tracheostoma valve or automatic speech valve, it has been experienced that these still do not hold the valve sufficiently to have a patient feel comfortable with it. Normally, the automatic speech valve is fixed to the patient's skin using a plaster or the like, however, when actuating the automatic speech valve, a great pressure results on that plaster, and eventually the plaster will come off.

The present invention seeks to provide an improved device for firmly fixing a tracheostoma valve of a laryngectomy patient

According to the present invention, an automatic tracheostoma speech valve fixation brace according to the preamble defined above is provided, comprising a fixation part for keeping an ATSV base element (e.g. in the form of an adhesive base plate) in position during use, and a holding part for holding the ATSV fixation brace to a patient, wherein at least a part of the holding part adjacent to the fixation part is made of a thermoplastic material, the thermoplastic material being moldable to the local anatomic shape of the patient, such as a patient's neck.

This allows to precisely mold the brace, which increases the wearing comfort, and provides a sufficiently strong fixation of the adhesive plaster on which the automatic speech valve is positioned, facilitating hands free speech. Even when using the fixation brace without using an actual ATSV, it can support the patient for manual speech. The present invention embodiments of the fixation brace contribute to the manual speech operations (use the patient's fingers to shut off the stoma), by better and more durably fixing the (ATSV) base element.

Further embodiments and alternatives with specific advantages are described with reference to the drawings below.

### Short description of drawings

The present invention will be discussed in more detail below, using a number of exemplary embodiments, with reference to the attached drawings, in which
Fig. 1 shows a front view of an automatic tracheostoma speech valve fixation brace according to an embodiment of the present invention,
Fig. 2 shows a front view of an automatic tracheostoma speech valve fixation brace according to a further embodiment of the present invention, and
Fig. 3 shows a simplified diagram of an automatic tracheostoma speech valve fixation brace worn by a patient.

### Detailed description of exemplary embodiments

The invention is defined in claim 1. In the present invention embodiments an automatic tracheostoma speech valve (ATSV) 9 is fixed on a patient with an ATSV base element 8, e.g. in the form of an ATSV plaster 8, the ATSV 9 and ATSV base element 8 being additionally supported by a fixation brace 1. The brace 1 assures that the ATSV base element 8 normally applied for holding the ATSV 9 to a patient is not stressed too much when the patient speaks. The brace 1 allows to take on the higher force exerted during speech by the patient on the ATSV 9 and from there on to the ATSV base element 8. The brace 1 according to the present invention embodiments is (partly) made of a special material allowing to shape the brace 1 to the (local) anatomic shape of the patient, such as an individual neck profile. The brace 1 is thus suitable for a large group of patients. As a result, the present brace embodiments can aid in improving the hands free speech of a patient who has undergone a total laryngectomy.

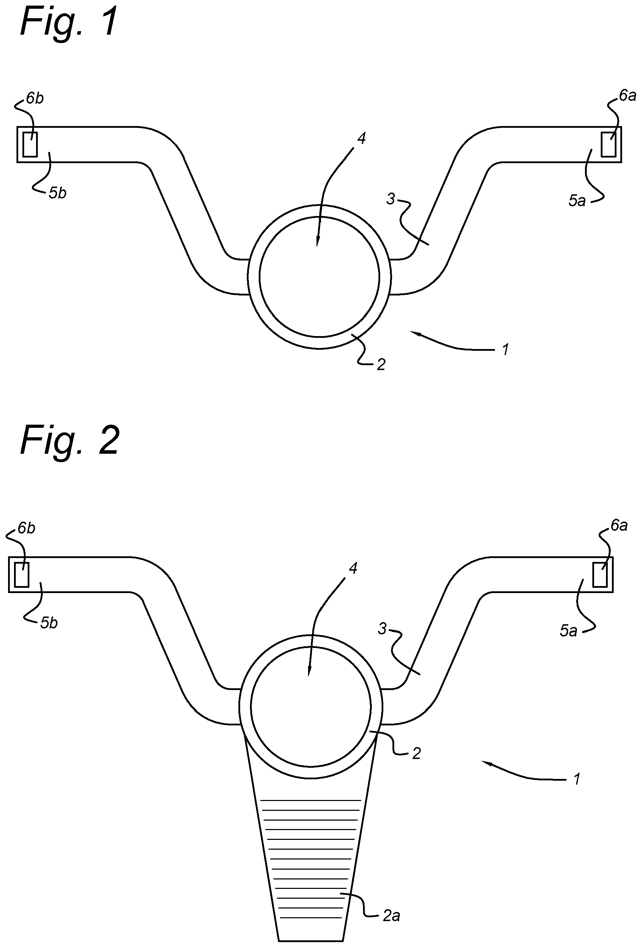

As shown in the schematic view of the embodiment shown in Fig. 1, the ATSV fixation brace 1 comprises a fixation part 2 for keeping an ATSV base element 8, and an ATSV 9 positioned therein in position during use, and a holding part 3 for properly holding of the ATSV fixation brace 1 to a patient, e.g. around a patient's neck. At least the holding part 3 (or at least the part thereof directly adjacent to the fixation part 2) comprises a thermoplastic material, the thermoplastic material being moldable to the local anatomic shape of the patient. This allows the ATSV fixation brace 1 to be adapted to the specific local anatomic shape of an individual patient, resulting in a better fit and more convenience and comfort for the patient. In a further embodiment, also the fixation part 2 is made of the same thermoplastic material, the thermoplastic material being moldable to the local anatomic shape of the ATSV base element 8 when in use by the patient..

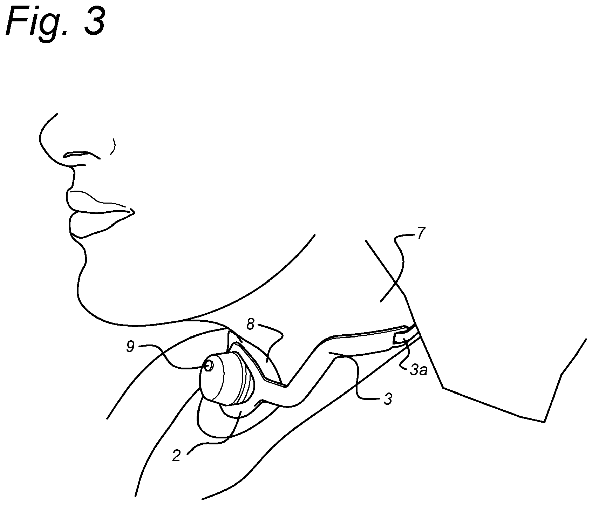

The brace 1 in the embodiment shown is provided with a holding part 3 with two sections on each side of the fixation part 2. The end parts 5a, 5b of each section are here provided with co-operating connection elements 6a and 6b respectively, e.g. in the form of Velcro parts. These connection elements 6a, 6b can be used to close the brace 1 around a patient's neck, e.g. using an additional band 3a (see Fig. 3). The connection elements 6a, 6b are attached to the end parts 5a, 5b of the brace 1 by heating the thermoplastic material locally, sufficiently for attaching the connection elements 6, e.g. by molding a part of the end parts 5a, 5b around the connection elements 6a, 6b. As an alternative, the thermoplastic material may be heated until it becomes sticky, allowing to glue the connection elements 6a, 6b to the end parts 5a, 5b.

As mentioned the connection elements 6a, 6b may be co-operating Velcro parts. Alternatively other mechanical connection elements, such as clips and hooks may be provided.

In a further specific embodiment, the holding part 3 is shaped to allow a good fixture of the brace 1 around a patient's neck without any further connection elements, e.g. by having the end parts 5a, 5b overlap over a (relatively) large distance, or by proper shaping of the holding part 3.

In a further embodiment, the brace 1 is provided with end parts 5a, 5b which do not span the entire patient's neck 7. In this embodiment, a further fixture, e.g. in the form of a flexible band is provided. The flexible band may also be provided as a band of which the length is adjustable.

In the embodiment shown, the fixation part 2 is provided with a (substantially) round opening 4 which can hold the ATSV 9 in position in an adhesive base element 8 of a patient. The fixation part 2 can have an opening which is adaptable to different sizes of tracheostoma valves, e.g. by using a thin and flexible inner ring part of the fixation part 2.

The fixation part 2 may be made of the same material as the holding part 3 as mentioned above, but furthermore this part may e.g. be made thicker thereby more readily keeping its shape in order to ensure a good fixation of the ATSV 9 in the ATSV base element 8. Alternatively, the shape of the fixation part 2 may be made to better withstand the forces applied on the ATSV base element 8 during speech, e.g. by having a tube form part. Furthermore, the fixation part may comprises a coating at an interior surface thereof, e.g. from stainless steel, in order to improve the ability to insert and remove the ATSV 9 from the ATSV base element 8.

As a major function of the fixation part 2 is to provide support to the ATSV base element 8, the diameter of the opening 4 can be slightly larger than the diameter of the ATSV 9 in a further embodiment.

In a further embodiment, which is shown schematically in Fig. 2, the ATSV brace 1 further comprises a chest extension part 2a, extending from the fixation part 2. This chest extension part 2a may also be made from a thermoplastic material, e.g. the same material as the rest of the ATSV brace 1. This chest extension part 2a may be attached to the patient's chest (e.g. using adhesive) and prevents the ATSV brace 1 from deformation (e.g. for a patient having a long neck).

Fig. 3 shows a simplified side view of a patient's neck 7, wearing a brace 1 according to one of the invention's embodiments for holding an ATSV 9. The part of the skin of a patient's neck 7 is provided with a tracheostoma plaster serving as ATSV base element 8. The holding part 3 is as shown formed to the anatomic shape of the patient's neck 7, e.g. closely following a collar bone curvature. In the embodiment shown, the fixation part 2 is keeping the ATSV 9 in position by virtue of the holding part 3, 3a fixing the brace 1 around the patient's neck 7.

Additionally, the fixation part 2 is in close contact with the plaster 8 by molding the fixation part after it is activated. Furthermore, such a close contact allows e.g. using adhesives known as such. In an embodiment, the fixation part 2 comprises a contact surface (back side of fixation part 2) for contacting and applying an adhesive, for firm contact with the stoma plaster 8 on the patient's skin.

In one embodiment, the thermoplastic material remains moldable for a first time period (or working time) after being heated to at least a first threshold temperature (or activation temperature). In other words, the thermoplastic material remains moldable at ambient room temperature, in contact with a person, for at least the first time period. This allows to bring the fixation brace 1 (holding part 3, or holding part 3 and fixation part 2) in a shape congruent with the local anatomic shape of the patient's body.

Thermoplastic material is a material which is rigid after cooled down to or being at ambient temperature for at least a cooling time period (or completion time). This allows the (anatomic) shape of the brace 1 to stay permanent, i.e. after a first fitting, the patient can remain using the brace 1 for a long period. Because of the specifically, individually shaped brace 1, the patient can wear the brace 1 with great comfort.

To further enhance the molding process and to enhance comfort during wearing, the thermoplastic material comprises a non-sticky surface, e.g. in the form of a coating, in a further embodiment. The non-sticky surface may be from a different material, or may result from proper treatment of the thermoplastic material.

The brace 1 can be made entirely from the same material. In one embodiment, the fixation part 2 and holding part 3 are integrally formed from a single sheet of the thermoplastic material. This makes fabrication of the brace 1 according to the present invention very easy and economical.

In one embodiment, the thermoplastic material is cut to shape, e.g. using laser cutting techniques. After tooling, the thermoplastic material either stays smooth on its own, or can be post-treated very easily.

The thermoplastic (memory) material used for the brace according to the present invention embodiments is e.g. a polycaprolactone (PCL) based material. This is a biodegradable polyester with a low melting point of around 60°C.

Once softened, it can be molded by hand in the proper shape. If the temperature of an outer layer decreases, it becomes non-sticky, but still pliable and moldable.

An example of such a thermoplastic material to be used is the Aquafit NS material commercially available from Orfit. This material is white when cooled down to ambient temperature, and is provided with a non-sticky layer (a coating dispersion of acrylat-urethane), allowing easier handling during molding of the brace. Activation can be easily monitored, as the color changes to transparent when reaching the activation temperature of about 65°C. This takes about 4-5 minutes when using heating in warm water. The working time of about 2 minutes is sufficient for molding the brace properly around a patient's neck. During a longer period (about 9 minutes, hardening time) the material is still flexible, but can no longer be molded. After about 25 minutes (completion time), the brace 1 is ready to be worn by a patient. Other similar materials may be used as well.

When the thermoplastic material for the brace 1 embodiments according to the present invention is reheated (i.e. heating to a temperature above a second threshold value), the material will regain its original shape (memory function), which allows to re-use the brace 1, or to re-initiate a proper molding and fitting procedure.

The material is very suited for application as a fixation brace 1, to support an adhesive base element 8 while using an ATSV 9, as it is scentless and has a smooth surface. No irritation of skin occurs during regular use.

As the material can be molded after heating above a threshold value, the brace 1 can be made suitable for almost every patient. After cooling down completely, the brace 1 retains its shape, as a result of which the fitting of the brace 1 is a simple and single event. It has been found that patients using the present brace 1 can speak for a longer period without hands (i.e. using the automatic tracheostoma speech valve 9 without holding a hand to it). Also it was experienced that speaking is more comfortable as a sort of resistance is being felt which holds the automatic speech valve 9 in position.

Furthermore, although a brace 1 is now needed to be worn, it was found that a patient is getting accustomed to this quite quickly. Whenever a patient is getting used to using an ATSV 9 with a brace 1 according to the present invention directly after a total laryngectomy, it was found that this present invention in one of its embodiments can be very helpful in speech revalidation of the patient.

The present invention embodiments have been described above with reference to a number of exemplary embodiments as shown in the drawings. Modifications and alternative implementations of some parts or elements are possible, and are included in the scope of protection as defined in the appended claims.

## Claims

1. Automatic tracheostoma speech valve (ATSV) fixation brace, comprising a fixation part (2) for keeping an ATSV base element (8) in position during use, and a holding part (3) for holding the fixation brace (1) to a patient around a patient's neck, **characterized in that** at least a part of the holding part (3) adjacent to the fixation part (2) is made of a thermoplastic material, wherein the thermoplastic material is a polycaprolactone based polymer, such that the thermoplastic material being moldable to the local anatomic shape of the patient.

2. Automatic tracheostoma speech valve fixation brace according to claim 1, wherein the fixation part (2) comprises a thermoplastic material, the thermoplastic material being moldable to the local shape of the ATSV base element (8) when in use by the patient.

3. Automatic tracheostoma speech valve fixation brace according to claim 1 or 2, wherein the fixation brace (1) further comprises a chest extension part (2a) extending from the fixation part (2).

4. Automatic tracheostoma speech valve fixation brace according to claim 1, 2 or 3, wherein the fixation part (2) comprises a tube form element.

5. Automatic tracheostoma speech valve fixation brace according to any one of claims 1-4, wherein the fixation part (2) comprises a coating at an interior surface thereof.

6. Automatic tracheostoma speech valve fixation brace according to any one of claims 1-5, wherein the thermoplastic material remains moldable for a first time period after being heated to at least a first threshold temperature.

7. Automatic tracheostoma speech valve fixation brace according to any one of claims 1-6, wherein the thermoplastic material is a material which is rigid after being cooled to ambient temperature for at least a cooling time period.

8. Automatic tracheostoma speech valve fixation brace according to any one of claims 1-7, the thermoplastic material is a material regaining its original shape after heating to a temperature above a second threshold value.

9. Automatic tracheostoma speech valve fixation brace according to any one of claims 1-8, wherein the thermoplastic material comprises a non-sticky surface.

10. Automatic tracheostoma speech valve fixation brace according to any one of claims 1-9, wherein the fixation part (2) and holding part (3) are integrally formed from a single sheet of the thermoplastic material.

11. Automatic tracheostoma speech valve fixation brace according to any one of claims 1-10, wherein the fixation part (2) comprises a contact surface for applying adhesive.

12. Automatic tracheostoma speech valve fixation brace according to any one of claims 1-11, wherein the fixation part (2) is adaptable to different size ATSV's.

13. Automatic tracheostoma speech valve fixation brace according to any one of claims 1-12, wherein the holding part (3) is provided with connection elements (6a, 6b), the connection elements (6a, 6b) being attached by heating the thermoplastic material locally.

14. Automatic tracheostoma speech valve fixation brace according to any one of claims 1-13, wherein the thermoplastic material is provided with a coating dispersion of acrylat-urethane.

## Patentansprüche

1. Fixierungsklammer für automatisches Tracheostoma-Sprechventil (ATSV), umfassend einen Fixierungsteil (2) zum In-Position-Halten eines ATSV-Basiselements (8) während der Verwendung und einen Halteteil (3) zum Halten der Fixierungsklammer (1) an einem Patienten um den Hals des Patienten herum, **dadurch gekennzeichnet, dass** wenigstens ein Teil des Halteteils (3), das neben dem Fixierungsteil (2) liegt, aus einem thermoplastischen Material besteht, wobei das thermoplastische Material ein Polymer auf Polycaprolactonbasis ist, so dass das thermoplastische Material an die lokale anatomische Form des Patienten angeformt werden kann.

2. Fixierungsklammer für automatisches Tracheostoma-Sprechventil gemäß Anspruch 1, wobei der Fixierungsteil (2) ein thermoplastisches Material umfasst, wobei das thermoplastische Material an die lokale Form des ATSV-Basiselements (8) angeformt werden kann, wenn es vom Patienten verwendet wird.

3. Fixierungsklammer für automatisches Tracheostoma-Sprechventil gemäß Anspruch 1 oder 2, wobei die Fixierungsklammer (1) weiterhin einen Brustkorbverlängerungssteil (2a) umfasst, der sich ausgehend vom Fixierungsteil (2) erstreckt.

4. Fixierungsklammer für automatisches Tracheostoma-Sprechventil gemäß Anspruch 1, 2 oder 3, wobei der Fixierungsteil (2) ein röhrenförmiges Element umfasst.

5. Fixierungsklammer für automatisches Tracheostoma-Sprechventil gemäß einem der Ansprüche 1 bis 4, wobei der Fixierungsteil (2) auf einer seiner Innenflächen eine Beschichtung umfasst.

6. Fixierungsklammer für automatisches Tracheostoma-Sprechventil gemäß einem der Ansprüche 1 bis 5, wobei das thermoplastische Material während einer ersten Zeitspanne formbar bleibt, nachdem es auf wenigstens eine erste Schwellentemperatur erhitzt wurde.

7. Fixierungsklammer für automatisches Tracheostoma-Sprechventil gemäß einem der Ansprüche 1 bis 6, wobei das thermoplastische Material ein Material ist, das hart ist, nachdem es während wenigstens einer Abkühlzeitspanne auf Umgebungstemperatur abgekühlt wurde.

8. Fixierungsklammer für automatisches Tracheostoma-Sprechventil gemäß einem der Ansprüche 1 bis 7, wobei das thermoplastische Material ein Material ist, das seine ursprüngliche Form zurückgewinnt, nachdem es auf eine Temperatur oberhalb eines zweiten Schwellenwerts erhitzt wurde.

9. Fixierungsklammer für automatisches Tracheostoma-Sprechventil gemäß einem der Ansprüche 1 bis 8, wobei das thermoplastische Material eine nichtklebrige Oberfläche umfasst.

10. Fixierungsklammer für automatisches Tracheostoma-Sprechventil gemäß einem der Ansprüche 1 bis 9, wobei der Fixierungsteil (2) und der Halteteil (3) aus einer einzigen Folie des thermoplastischen Materials einstückig ausgebildet sind.

11. Fixierungsklammer für automatisches Tracheostoma-Sprechventil gemäß einem der Ansprüche 1 bis 10, wobei der Fixierungsteil (2) eine Kontaktfläche zum Auftragen des Klebers umfasst.

12. Fixierungsklammer für automatisches Tracheostoma-Sprechventil gemäß einem der Ansprüche 1 bis 11, wobei der Fixierungsteil (2) an ATSVs unterschiedlicher Größe anpassbar ist.

13. Fixierungsklammer für automatisches Tracheostoma-Sprechventil gemäß einem der Ansprüche 1 bis 12, wobei der Halteteil (3) mit Verbindungselementen (6a, 6b) versehen ist, wobei die Verbindungselemente (6a, 6b) dadurch befestigt werden, dass man das thermoplastische Material lokal erhitzt.

14. Fixierungsklammer für automatisches Tracheostoma-Sprechventil gemäß einem der Ansprüche 1 bis 13, wobei das thermoplastische Material mit einer Beschichtungsdispersion aus Acrylat-Urethan versehen ist.

## Revendications

1. Orthèse de fixation d'une valve phonatoire de trachéostomie automatique (VPTA), comprenant une pièce de fixation (2) destinée à maintenir un élément de base VPTA (8) en position pendant l'utilisation, et une pièce de maintien (3) destinée à maintenir l'orthèse de fixation (1) sur un patient autour d'un cou du patient, **caractérisée en ce qu'**au moins une partie de la pièce de maintien (3) adjacente à la pièce de fixation (2) est en matière thermoplastique, dans laquelle la matière thermoplastique est un polymère à base de polycaprolactone, de sorte que la matière thermoplastique peut être moulée à la forme anatomique locale du patient.

2. Orthèse de fixation d'une valve phonatoire de trachéostomie automatique selon la revendication 1, dans laquelle la pièce de fixation (2) comprend un matériau thermoplastique, le matériau thermoplastique pouvant être moulée à la forme locale de l'élément de base VPTA (8) lorsqu'il est utilisé par le patient.

3. Orthèse de fixation d'une valve phonatoire de trachéostomie automatique selon la revendication 1 ou 2, dans laquelle l'orthèse de fixation (1) comprend en outre une partie d'extension de poitrine (2a) s'étendant depuis la pièce de fixation (2).

4. Orthèse de fixation d'une valve phonatoire de trachéostomie automatique selon la revendication 1, 2 ou 3, dans laquelle la pièce de fixation (2) comprend un élément de forme tubulaire.

5. Orthèse de fixation d'une valve phonatoire de trachéostomie automatique selon l'une quelconque des revendications 1 à 4, dans laquelle la pièce de fixation (2) comprend un revêtement sur une surface intérieure de celle-ci.

6. Orthèse de fixation d'une valve phonatoire de trachéostomie automatique selon l'une quelconque des revendications 1 à 5, dans laquelle la matière thermoplastique peut être moulée pendant un premier intervalle de temps après avoir été chauffée à au moins un premier seuil de température.

7. Orthèse de fixation d'une valve phonatoire de trachéostomie automatique selon l'une quelconque des revendications 1 à 6, dans laquelle la matière thermoplastique est une matière qui est rigide après avoir été refroidie à la température ambiante pendant au moins un intervalle de temps de refroidissement.

8. Orthèse de fixation d'une valve phonatoire de trachéostomie automatique selon l'une quelconque des revendications 1 à 7, la matière thermoplastique est une matière reprenant sa forme initiale après chauffage à une température supérieure à une deuxième valeur de seuil.

9. Orthèse de fixation d'une valve phonatoire de trachéostomie automatique selon l'une quelconque des revendications 1 à 8, dans laquelle la matière thermoplastique comprend une surface non collante.

10. Orthèse de fixation d'une valve phonatoire de trachéostomie automatique selon l'une quelconque des revendications 1 à 9, dans laquelle la pièce de fixation (2) et la pièce de maintien (3) sont intégralement formées à partir d'une seule feuille de la matière thermoplastique.

11. Orthèse de fixation d'une valve phonatoire de trachéostomie automatique selon l'une quelconque des revendications 1 à 10, dans laquelle la pièce de fixation (2) comprend une surface de contact destinée à l'application d'un adhésif.

12. Orthèse de fixation d'une valve phonatoire de trachéostomie automatique selon l'une quelconque des revendications 1 à 11, dans laquelle la pièce de fixation (2) est adaptable à différentes tailles du VPTA.

13. Orthèse de fixation d'une valve phonatoire de trachéostomie automatique selon l'une quelconque des revendications 1 à 12, dans laquelle la pièce de maintien (3) est pourvue d'éléments de connexion (6a, 6b), les éléments de connexion (6a, 6b) étant fixés par chauffage local de la matière thermoplastique.

14. Orthèse de fixation d'une valve phonatoire de trachéostomie automatique selon l'une quelconque des revendications 1 à 13, dans laquelle la matière thermoplastique est pourvue d'une dispersion de revêtement en acrylate-uréthane.
